# EUROPEAN PATENT APPLICATION

(11) **EP 1 005 901 A2**
(43) Date of publication of application: **07.06.2000**
(21) Application number: 00101315.0
(22) Date of filing: 12.11.1996
(51) Int. Cl.: B01F 13/00, B01F 13/06, A61F 2/46

(54) **Method and device for mixing components for bone cement in a mixing vessel**

(30) Priority: 13.11.1995 US 545591; 22.10.1996 US 734817
(62) Divisional of application: 96939409.7
(71) Applicant: CEMVAC SYSTEM AKTIEBOLAG, 589 41 Linköping (SE)
(72) Inventor: Jonsson, Sören, S-589 41 Linköping (SE)
(74) Representative: Willquist, Bo

(57) **Abstract**

An apparatus for mixing a liquid and a powder component (A, B) in a mixing vessel (2) for the preparation of a bone cement. The mixing vessel (2) interior (2a) is maintained under a vacuum in order to prevent harmful emissions from escaping from said vessel once said liquid and powder components (A, B) are mixed. The mixing vessel (2) is pre-filled with the powder component (B) and is defined by an outer wall (3) having a top end and a bottom end. The top end is formed with a sealable spout (5), said bottom end formed with an axially displaceable bottom (4). The apparatus comprises:
A means (1) for introducing the liquid component (A) into the mixing vessel (2);
An agitator (6) is comprised of a tubular rod (6b) which extends upwardly out of the interior of the vessel through said spout (5) and an agitator disk (6a) is attached to the tubular rod (6b). First open end of the tubular rod defining a mouth and a second open, end of the tubular rod is encircled by the disk (6a), the tubular rod (6b) being axially displaceable within the vessel (2) interior for mixing the bone cement components (A, B);
A removable tightening rod (19) is disposed within the tubular rod (6b) for sealing the open bottom rod end from communication with the atmosphere after the liquid component (A) is introduced into the mixing vessel (2), and being disposed within the tubular rod (6b) prior to introducing the liquid (A) into said mixing vessel (2);
Said means (1) for introducing component liquid (A) into the vessel (2) is comprised of a container (9) having an interior for containing component (A), a tip (9c) and a tube (26) conntected to said tip. The tube (26) has one end inserted into the outer wall (3) of the mixing vessel (2).

The powder (B) and liquid (A) components are then mixed within the vessel (2) under a continuous vacuum the atmospheric air is prevented from entering into the vessel (2) due to said tightening rod (19), wherein said harmful emissions caused from mixing the components (A, B) are prevented from escaping said vessel (2).

## Description

### BACKGROUND OF THE INVENTION

### Field of the Invention

The present invention relates to a method for mixing constituent components in a mixing vessel for the preparation of bone cement under vacuum. The invention also relates to an apparatus mixing constituent components in a mixing vessel under partial vacuum for the preparation of bone cement.

### Description of the Prior Art

Bone cement is prepared by mixing polymethyl methacrylate in powder form, with liquid monomethyl methacrylate in a mixing container. Both the liquid component and the combined mixture give off substances in gaseous form which are environmentally harmful and injurious to the health. For this reason, it is important for the introduction of the bone cement components into the mixing container and the mixing process itself, to take place in such a way that the smallest possible quantity of the harmful gases escape into the surrounding environment. Mixing vessels in which the introduced components were successfully prepared into bone cement without a substantial release of the aforementioned gases are described in SE-C-8901599-4 and SE-A0-9201353-1 and WO 94/26403, for example. In order for the bone cement to develop optimal strength during use, it is also important for the components comprising the cement to have well-mixed, predetermined portions.

In said WO-publication a glass ampoule containing a liquid component of a bone cement is surrounded by a container which is in reclosable communication with the atmosphere. A second container surrounds the first container so that when the mixing vessel is opened, the contents of the ampoule, under the effect of a partial vacuum inside the mixing vessel, can be sucked down into it, a space formed between the aforementioned inner container and outer container is filled with a second bone cement component in powder form which is caused by displacement of the inner container relative to the outer container, to move from a first position in which the space does not communicate with the atmosphere or the mixing vessel to a second position in which the space communicates with the atmosphere and the mixing vessel, so that the powdered bone cement component is sucked down into the mixing vessel under the effect of the partial vacuum inside it.

A device for carrying out the above method which device is also explained in said WO-publication is characterized in that it comprises, an inner container communicating with the atmosphere, and is so arranged as to enclose the glass ampoule containing the liquid bone cement component, and to communicate with the aforementioned mixing vessel, and which includes means for opening the ampoule so that its contents, under the effect of the partial vacuum inside the mixing vessel, can be sucked down into it. The outer container at least partially encloses the inner container and is also arranged so as to communicate with the mixing vessel and together with the inner container, defines a space therebetween which is filled with a certain quantity of the powdered component of bone cement. The inner container is capable of displacement from a first position to a second position, the first position characterized by the inner container preventing communication between both the mixing vessel and the atmosphere, and the second position characterized, in which communication between the mixing vessel on the one hand and the atmosphere on the other hand is open, so that the bone cement component in powder form, under the effect of the partial vacuum inside the mixing vessel, can be sucked into it without escape of gases.

### SUMMARY OF THE INVENTION

One object of the present invention is to make available a method and device of the kind described above, which avoids the risk of gas release when feeding the bone cement components into the mixing vessel. Another object is to make the mixing as convenient and simple as possible for a person using the device. Still another object is to make the mixing as safe as possible for said person i.e. reduce to a minimum the failure rate of mixing. This is achieved in accordance with the apparatus and method of the invention.

A first method of the present invention simply involves a providing a glass ampoule with the liquid component therein, breaking the ampoule, and then supplying the contents into the mixing vessel via a tubing through an opening in the cylindrical wall of the vessel. The liquid component is sucked under vacuum into the mixing vessel.

According to a second method of the present invention, the liquid bone cement component is supplied through a collapsible plastic bag and tubing arrangement attached thereto. The tubing can be connected to the mixing vessel through the cylindrical wall of the vessel, where a tubing clamp is released to allow the liquid component to flow into the mixing vessel under the influence of vacuum.

The devices for carrying out the methods in accordance with this invention are more fully detailed in the following sections.

### BRIEF DESCRIPTION OF THE DRAWINGS

The invention is described below in greater detail with reference to the accompanying drawing, in which:
Figure 1 illustrates the mixing vessel of a first embodiment of the present invention in which the liquid form of the bone cement is fed into the mixing vessel through the side wall;
Figure 2 illustrates the mixing vessel of a second embodiment of the present invention where the liquid bone cement component is contained in a collapsible plastic bag.

### DETAILED DESCRIPTION OF THE INVENTION

The designations 1 and 2 are used generally in the drawing in respect of a feed arrangement and a mixing vessel. The latter comprises an interior 2a, a cylindrical container 3 comprised of an outer cylinder wall 3a, a bottom 4 at one end of the container and a spout 5 with sealed opening at the other end, together with an agitator 6, received within said spout and mixing vessel and capable of axial, vertical movement inside the container 3. The agitator 6 consists of an agitator disc 6a attached to a tubular agitator rod 6b. The agitator 6 is mounted so that it is free to vertically slide up and down while maintaining a seal in the spout 5, in such a way that the plurality of holes 6h in agitator disk 6a can be used to bring about through mixing of the bone cement components A, B within mixing vessel 2 with no gaseous escape to atmosphere. Once mixing is complete, and once a lock 7 has been removed, the bottom 4 can be axially displaced inside the cylinder by upward movement of piston head 80, moving towards the spout 5. The pistonlike function of bottom 4, upwardly pushes and then discharges the mixed bone cement via the hollow agitator rod 6b, which now serves as a discharge nozzle. The interior of the container 2 communicates via a filter 8 with a vacuum source (not shown) during feeding and mixing of the bone cement components A, B. Rapid and effective feeding of the bone cement components into the mixing vessel, and safe handling of the gases that are environmentally harmful and injurious to human health, is achieved in this way.

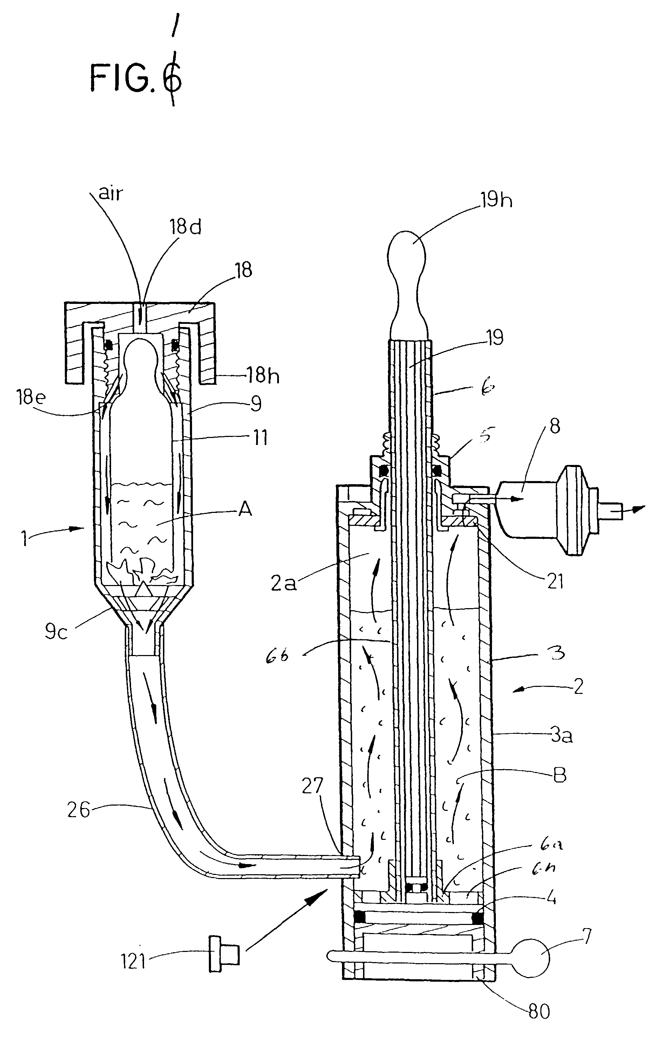

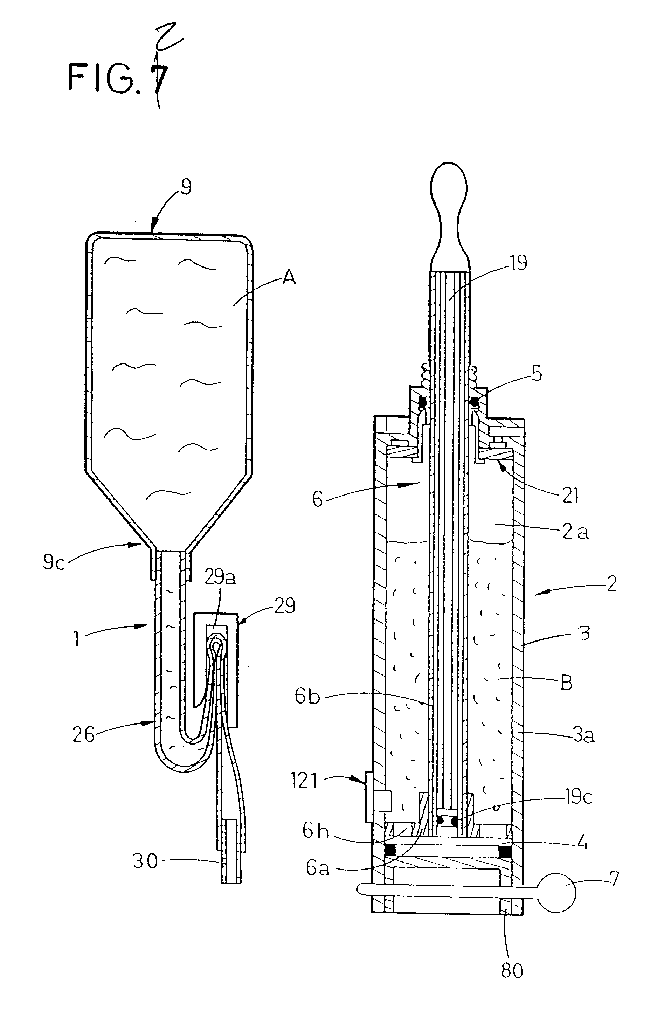

Turning attention now to Figures 1 and 2, a first and a second embodiment of the present invention will now be described. The powdered component B of bone cement pre-exists within the mixing vessel 2 prior to any mixing procedures, and the container 9 contains only the liquid component A. It will become clearer after reading the following description that the main characteristic of the first embodiment is that the liquid component A will be drawn into the mixing vessel under the influence of vacuum. The first embodiment uses an ampoule arrangement where the contents enter through the outer cylindrical wall 3a. The second embodiment uses a plastic container which by means of a tubing is connected to the cylindrical wall of the mixing chamber. The first and second embodiments are also provided with a second filter 21, located at the top of the mixing vessel 2.

In accordance with the first embodiment, Figure 1 shows mixing vessel 2 as being pre-filled with the powder component B of the bone cement. A tightening rod 19 is received within tubular agitator rod 6b of agitator 6 and has plug 19a and O-ring 19c inserted within a groove 19b thereof, to form an air-tight seal so that powdered contents B are not contacted by and affected by atmospheric air which is capable of downwardly travelling along tubular rod 6b to vessel bottom 4.

Figure 1 shows the first embodiment of the present invention wherein the mixing vessel as mentioned is pre-filled with powder component B and where container 9 has bottom end 9c connected to a tube 26, shown as being inserted into hole 27 which penetrates cylinder wall 3a. It is to be understood that prior to insertion of tube 26, plug 121 is inserted within hole 27, thereby maintaining a seal from the atmosphere. When mixing is to take place the interior of the mixing vessel 2 is put under vacuum by starting the vacuum source, not shown. Then plug 121 is removed, tube 26 is inserted into hole 27 and the glass ampoule containing liquid A is broken. By percolation of air and liquid A through powder component B in mixing vessel 2 a pre-mixing is obtained. When the ampoule is emptied tube 26 is removed from hole 27, plug 121 is inserted and final mixing by means of the agitator is performed. After finished mixing lock 7 is removed and the contents pushed upwards with piston head 80 for eventual discharge out tube 6b.

Figure 2 shows a second embodiment, where container 9 is now comprised of collapsible plastic bag. This substitution advantageously reduces the cost to manufacture, and is less bothersome than breaking and discarding the glass ampoule bottles. Again, this arrangement functionally mixes the elements together as previously explained. However, as seen in Figure 2, a U-shaped sleeve member 29 is used as a valve, where tube 26 is folded and frictionally inserted within sleeve interior 29a, thereby blocking any flow of material A. Then, plug 121 is removed from cylindrical wall 3a, and is inserted into hole 27. Figure 2 shows a coupling 30 being inserted into tube 26 to facilitate the connection into the cylinder wall to allow discharge of liquid A into mixing vessel 2. It should be realized that the embodiments of Figure 1 could also be provided with coupling 30 if desired. Mixing is completed by upwardly and downwardly moving agitator disk 6a as previously described, using mouth 6c of rod 6b as discharge nozzle once desk 6a is contacted against top end 3a of cylinder 3, and tightening rod 19 is removed so that the mixed contents can be pushed upwards by bottom 4 through hollow agitator 6 after that tightening rod 19 has been removed.

## Claims

1. An apparatus for mixing a liquid and a powder component (A, B) in an interior of a mixing vessel (2) for the preparation of a bone cement, said mixing vessel (2) interior (2a) maintained under a vacuum created from a vacuum source in order to prevent harmful emissions from escaping from said vessel once said liquid and powder components (A, B) are mixed, comprising:
a mixing vessel (2) pre-filled with a powder component (B) of said bone cement said vessel (2) defined by an outer wall (3) having a top end, a bottom end and an interior, said top end formed with a sealable spout (5), said bottom end formed with an axially displaceable bottom (4);
a means (1) for introducing said liquid component (A) into said interior of said mixing vessel (2);
an agitator (6) received within said vessel interior (2a), said agitator (6) comprised of a tubular rod (6b) which extends upwardly out of said interior through said spout (5) and is in communication with the atmosphere and an agitator disk (6a) attached to said tubular rod (6b), an open, first end of said tubular rod defining a mouth and an open, second end of said tubular rod encircled by said disk (6a), said tubular rod (6b) axially displaced within said vessel (2) interior for mixing said bone cement components (A, B);
a removable tightening rod (19) disposed within said tubular rod (6b) for sealing said open bottom rod end from communication with the atmosphere after said liquid component (A) is introduced into said mixing vessel (2), said tightening rod (19) being disposed within said tubular rod (6b) prior to introducing said liquid (A) into said mixing vessel (2);
wherein said means (1) for introducing said liquid (A) into said vessel (2) is comprised of a container (9) having an interior for containing said liquid (A), a tip (9c) and a tube (26) connected to said tip, said tube (26) having one end inserted into said outer wall (3) of said mixing vessel (2), whereby harmful emissions are prevented from escaping the vessel (2) during introduction of said liquid component (A) due to the vacuum present in said vessel (2) wherein said powder (B) and liquid (A) components are then mixed within said vessel (2) interior (2a) under continuous vacuum from said vacuum source and said atmospheric air is prevented from entering into said vessel (2) due to said tightening rod (19), wherein said harmful emissions caused from mixing said components (A, B) are prevented from escaping said vessel (2).

2. The apparatus of Claim 1, wherein the tube (26) is inserted into said outer wall (3) of the mixing vessel (2) near said bottom end.

3. The apparatus of Claim 1 wherein said plastic tube (26) is provided with a clip (29) for opening and closing said tube (26) upon demand.

4. The apparatus of Claim 1 wherein said container is comprised of a glass ampoule.

5. The apparatus of Claim 1 wherein said container is comprised of a collapsible plastic bag.

6. A method for mixing a liquid and a powder bone cement component (A, B) in a mixing vessel (2) maintained under vacuum for the preparation of said bone cement wherein said mixing vessel (2) is provided with a pre-determined amount of said powder component (B) of said cement, the method comprising the steps of providing a mixing vessel (2) which said vessel is defined by a cylindrical cylinder having an open interior (2a) with a spout (5) attached to one end of said cylinder, and having an axially displaceable bottom (80);
inserting a mixing agitator (6) within said spout (5) so as to communicate with said vessel interior (2a), said agitator (6) comprised of a tubular rod (6b) having an agitator disk (6a) fixed on one end thereof, said other end being open and defining a mouth, said mouth being located axially above said spout of said vessel, said agitator (6) axially displaced such that said agitator disk (6a) can mix both of said bone cement components (A, B) together;
providing a tightening rod (19) within said tubular rod (6b) so as to seal said vessel (2a) from said atmosphere;
providing a hole in said container (9) wall and connecting a tube between said container (9) and vessel (2) in order to feed said liquid (A) through said tube to said container;
introducing said liquid component (A) into said interior of said vessel (2a);
axially displacing said agitator (6) so as to mix said liquid and powder components (A, B) under vacuum, without allowing harmful emissions to escape said mixing vessel.

7. The method of Claim 6 wherein said container (9) is a plastic bag containing said liquid (A) and said container (9) is provided with a tube that connects said container with the vessel wall in order to feed said liquid (A) into said vessel (2).

8. The method of Claim 7, wherein said liquid component (A) is fed into said vessel (2) near said bottom (80).
